# EUROPEAN PATENT SPECIFICATION

(11) **EP 2 459 245 B1**
(45) Date of publication and mention of the grant of the patent: **14.08.2013**
(21) Application number: 10737829.1
(22) Date of filing: 14.07.2010
(51) Int. Cl.: A61M 1/02

(54) **DEVICE FOR EXTRACTING BLOOD FROM THE PLACENTA AND/OR UMBILICAL CORD**
VORRICHTUNG ZUR BLUTEXTRAKTION AUS DER PLAZENTA UND/ODER NABELSCHNUR
DISPOSITIF POUR EXTRAIRE LE SANG DU PLACENTA ET/OU DU CORDON OMBILICAL

(30) Priority: 30.07.2009 ES 200930536
(43) Date of publication of application: 06.06.2012
(73) Proprietor: Álvarez Ramos, Ángel Gabriel, 33201 Gijón (ES)
(72) Inventor: Álvarez Ramos, Ángel Gabriel, 33201 Gijón (ES)
(74) Representative: Illescas, Manuel
(86) International application number: PCT/EP2010/060131
(87) International publication number: WO 2011/012449

(56) References cited:
- WO-A1-92/03180
- WO-A1-98/07461
- WO-A2-2005/041772
- US-A- 2 572 314
- US-A- 3 982 546

## Description

### FIELD OF THE INVENTION

The present invention relates to a device designed to simultaneously extract and collect blood from the placenta and/or umbilical cord, and more particularly, to a device based on collecting said blood while it falls through the effect of gravity, in combination with a suction system to facilitate its extraction.

### BACKGROUND OF THE INVENTION

In recent years, the discovery of the existence of high stem cell content in the umbilical cord and placenta of pregnant women, has favoured the development of various systems for extracting and collecting the blood contained in said organs, with a view to storing it for subsequent use, or for scientific purposes, or for use, in case of need, for transplantation to patients suffering from various congenital or acquired diseases, such as immune deficiencies, cancerous diseases or metabolic disorders related to deposits, among others.

For transplants of blood from the umbilical cord and placenta to be effective, providing the therapeutic level required by the treated pathologies, it is of crucial importance to have a sufficient volume of stem cell-rich blood, requiring, at least, approximately 1.5x10⁷ of precursor cells per Kg of the patient's weight for a successful implantation.

Currently, the majority of systems aimed at extracting blood from the umbilical cord and placenta, are based on mechanisms that pierce the umbilical cord, immediately after delivery of the baby, in the minutes prior to the expulsion of the placenta. Blood thereby extracted, falls through gravity, into a sack that collects the blood, said sack being held below the height of the woman who has just given birth, in such a way as to encourage the drop of blood. Normally, the sack contains anticoagulants and is kept in motion during the collection process, favouring the blood's preservation in optimum conditions.

By means of the foregoing method, the average volume of blood collected from each labour is estimated at approximately 90 ml. Although, in many cases, this volume is sufficient to obtain a stem cell content that a posteriori may be therapeutically effective, it also happens in a non-negligible number of patients that said volume does not manage to provide the required content in stem cells, due to insufficient cellularity.

Currently, the state of the art contemplates various systems designed to increase the amount of placental and umbilical cord blood extracted and collected, fundamentally using pressurised systems that allow said blood to be pumped, increasing thereby the collected volume and reducing the extraction time. Some examples of this type of system are the devices described in patents WO2005/041772, US5097842, US5059168 and US5575795. Said devices comprise one or more needles to pierce the umbilical cord and/or placenta, blood collection means, such as reservoirs, sacks, or vacuum containers, and suction or drainage means, such as vacuum pumps, peristaltic pumps, or syringes. In all of the references mentioned above, the amount of blood collected is increased by replacing the usual method, based on puncture and subsequent fall by gravity, with a method based exclusively on the negative pressure exerted by the suction or drainage means.

Although the aforesaid devices are capable of achieving the purposes for which they are designed, producing an increase in the amount of blood collected, they share a common problem related to their designed based on a suction method. As a result of continuously applying said suction, there is a possibility of precipitating the appearance of complications such as the sudden appearance of retroplacentary hematoma and, consequently, premature expulsion of the placenta before the necessary time has passed for said expulsion to occur naturally and for the maternal haemostasis factors that follow birth minimising the haemorrhage. This expulsion, which may be precipitated by an uncontrolled constant suction of blood, represents an obvious risk to the mother's health.

Therefore, it is necessary to develop devices that make it possible to increase the volume of blood collected from the placenta and umbilical cord, in such a way that the content of extracted stem cells is sufficient to fulfil the required therapeutic purposes, and that, at the same time, are safe for the mother in the phase immediately after giving birth, guaranteeing the natural expulsion of the placenta at no health risk whatsoever.

The invention is defined by the features of independent claim 1. The present invention is aimed at satisfying this need, by means of an extraction device that combines the usual system of blood falling by gravity, with the controlled assistance provided by a suction system.

Another advantage of the present invention is that, through a design that combines extraction by gravity and extraction by suction, it is possible to extract the blood contained in both the umbilical cord as well as the placenta by means of piercing only the umbilical cord. Not having to pierce the placenta is particularly relevant, since said puncture normally entails a risk of bacterial contamination avoided with the device described herein. Additionally, the possibility of extracting blood from the placenta by piercing the umbilical cord, makes it possible to extract blood immediately after the baby's delivery, without having to wait for the placenta to be expelled, which normally occurs within 5 and 30 minutes after giving birth, representing an optimisation in working times of the operating theatre, with the ensuing cost savings.

### OBJECT OF THE INVENTION

One object of the present invention is a device for extracting blood from the placenta and umbilical cord which simultaneously combines collecting blood as it falls due to the effect of gravity, with a suction system that facilitates said fall.

One object of the present invention is a device for extracting blood from the placenta and umbilical cord which comprises, at least, one blood extraction means consisting of, at least, one needle, catheter, or similar and at least one tube, pathway or similar, through which the extracted blood circulates, wherein each tube is connected to, at least, one means for collecting blood, said blood falling into the collection means by the effect of gravity and the collection means being connected to, at least, one suction means.

One object of the present invention is a device for extracting blood from the placenta and umbilical cord which allows the aforesaid extraction to be carried out by piercing the umbilical cord without having to make any puncture in the placenta.

In a preferred embodiment of the present invention, the blood extraction means include, at least one needle, catheter or similar and a tube, pathway or similar as backup. In this way it is possible to have a backup system directly attached to the extraction device, for use in case of need, without having to replace any additional element, which could delay or complicate the extraction process.

In a preferred embodiment of the present invention, at least one of the tubes has a closing mechanism, preferably through clamp-type grips, or alternatively valve or by-pass systems, manually or automatically actuated. This provides means for controlling and retaining the extracted blood.

In a preferred embodiment of the present invention, the collection means contain anticoagulants inside. This allows the extracted blood to be preserved in optimum conditions.

In a preferred embodiment of the present invention, the suction means can be operated manually or automatically. This provides an adaptable device for varying functionalities, according to the requirements of each extraction process.

Other characteristics and benefits of the present invention will be inferred from the following description, as well as the illustrative embodiment of the accompanying drawing.

### DESCRIPTION OF THE DRAWINGS

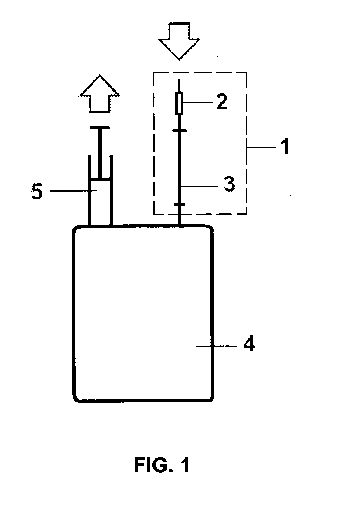

Figure 1 is a schematic representation of an embodiment of the device described by the present invention.

### DETAILED DESCRIPTION OF THE INVENTION

In accordance with the invention, and following the embodiment represented in Figure 1, the device for extracting and collecting blood consists of, at least, one blood extraction means (1) which comprises, preferably, at least, one needle, catheter or similar (2) designed to pierce the umbilical vein and/or venous system of the placenta and, at least, one tube, pathway or similar (3) through which the extracted blood circulates. In different embodiments of the present invention, it is possible to have more than one tube and needle as a backup system, in case of need. In another preferred embodiment, the tube (3) has a closing mechanism, preferably, through clamp-type grips. Each tube (3) is connected to, at least, one blood collection means (4), which preferably consists of a collection sack incorporating anticoagulants inside in order to preserve the blood. Each collection means (4) is connected, in turn, to at least one suction means (5), which consists of a plunger system, manually or automatically operated.

The procedure for extracting blood carried out by means of the present invention therefore comprises the following stages:
a) Piercing the umbilical vein and/or venous system of the placenta with the needle (2). Optionally, the umbilical vein can be pierced to extract the blood contained in the placenta, without having to puncture the latter.
b) Keeping the collection means (4) at a height below that of the mother and in constant motion, favouring, respectively, the falling of blood through the effect of gravity and keeping the blood in optimum conditions.
c) Using the suction means (5) to facilitate the blood's extraction and fall due to gravity into the collection means (4), in a controlled manner, applying a sufficiently gentle negative pressure so as to prevent expulsion of the placenta, said pressure being evaluated by the doctor, according to the inherent characteristics of each patient, as well as the circumstances of the delivery.

By means of the described process it is possible therefore, to carry residual blood existing in the placenta and umbilical cord, which is not accessible using a method based exclusively on the fall by gravity. This allows the volume of blood collected to be increased significantly, said increase being estimated as being within the range of 80 to 100 ml, corresponding to an increase of between 89% and 111% against the volume collected by the usual methods based on fall due to gravity.

Additionally, the existence of suction means present in the device of the invention, which can be operated in a gentle and controlled manner and designed as a support to the method for extracting and collecting blood by gravity, is capable of providing the guarantee that the placenta is not expelled prematurely, thereby preventing the risk to the mother's health associated to the methods of extraction through suction existing in the state of the technique.

All described embodiments for the present invention must not be considered as limiting against other variations in its design or materials used in its manufacture, on condition that such variations do not alter the essence of the invention in addition to the object thereof.

## Claims

1. Device for extracting blood from the placenta and/or umbilical cord, comprising at least, one blood extraction means (1) consisting of, at least, one needle or catheter (2) suitable for piercing the umbilical vein and/or the venous system of the placenta and, at least, one tube or pathway (3) through which the extracted blood circulates, wherein each tube or pathway (3) is connected to, at least, one blood collection means (4), said blood dropping into the collection means (4) through the effect of gravity, and **characterised in that** said collection means (4) is connected to, at least, one suction means (5) comprising a plunger system.

2. Device for extracting blood according to claim 1, **characterised in that** the blood extraction means (1) include, additionally, at least, one backup needle (2) and tube (3).

3. Device for extracting blood according to any of claims 1-2, **characterised in that** some of the tubes (3) have, at least, a closing mechanism, preferably, using clamp-type grips.

4. Device for extracting blood according to any of claims 1-3, **characterised in that** the collection means (4) contain, inside, anticoagulants.

5. Device for extracting blood according to any of claims 1-4, **characterised in that** the suction means (5) can be operated manually or automatically.

## Patentansprüche

1. Vorrichtung zur Blutentnahme aus der Plazenta und/oder der Nabelschnur, die mindestens ein Blutentnahmeset (1) umfasst, das aus mindestens einer zum Einstechen in die Nabelvene und/oder in das Venensystem der Plazenta geeigneten Nadel oder Katheter (2) und mindestens einem Röhrchen oder Kanal (3), durch das das entnommene Blut fließt, besteht, wobei jedes Röhrchen oder Kanal (3) mit mindestens einem Blutsammelbehälter (4) verbunden ist und besagtes Blut unter Einwirkung der Schwerkraft in den Sammelbehälter (4) tropft, **dadurch gekennzeichnet, dass** besagter Sammelbehälter (4) mit mindestens einem ein Kolbensystem umfassenden Saugelement (5) verbunden ist.

2. Vorrichtung zur Blutentnahme gemäß Anspruch 1, **dadurch gekennzeichnet, dass** das Blutentnahmeset (1) zusätzlich mindestens eine Ersatznadel (2) und -röhrchen (3) einschließt.

3. Vorrichtung zur Blutentnahme gemäß den Ansprüchen 1 bis 2, **dadurch gekennzeichnet, dass** einige der Röhrchen (3) mindestens einen Schließmechanismus aufweisen, bei dem vorzugswiese klammerartige Spangen verwendet werden.

4. Vorrichtung zur Blutentnahme gemäß einem der Ansprüche 1 bis 3, **dadurch gekennzeichnet, dass** der Sammelbehälter (4) in seinem Innern einen Gerinnungshemmer enthält.

5. Vorrichtung zur Blutentnahme gemäß einem der Ansprüche 1 bis 4, **dadurch gekennzeichnet, dass** das Saugelement (5) manuell oder automatisch betrieben werden kann.

## Revendications

1. Dispositif pour extraire du sang du placenta et/ou du cordon ombilical, comprenant au moins, un moyen d'extraction de sang (1) constitué, au moins, d'une aiguille ou d'un cathéter (2) adapté pour percer la veine ombilicale et/ou le système veineux du placenta et, au moins, un tube ou circuit (3) à travers lequel le sang extrait circule, dans lequel chaque tube ou circuit (3) est relié à, au moins, un moyen de collecte de sang (4), ledit sang tombant dans le moyen de collecte de sang (4) sous l'effet de la gravité, et **caractérisé en ce que** ledit moyen de collecte de sang (4) est relié à, au moins, un moyen d'aspiration (5) comprenant un système de piston.

2. Dispositif pour extraire du sang selon la revendication 1, **caractérisé en ce que** les moyens d'extraction de sang (1) comprennent, en outre, au moins, une aiguille (2) et un tube (3) de réserve.

3. Dispositif pour extraire du sang selon n'importe laquelle des revendications 1-2, **caractérisé en ce que** certains des tubes (3) possèdent, au moins, un mécanisme de fermeture, de préférence, utilisant des serrages de type clamp.

4. Dispositif pour extraire du sang selon n'importe laquelle des revendications 1-3, **caractérisé en ce que** des anticoagulants sont contenus à l'intérieur des moyens de collecte (4).

5. Dispositif pour extraire du sang selon n'importe laquelle des revendications 1-4, **caractérisé en ce que** les moyens d'aspiration (5) peuvent être actionnés manuellement ou automatiquement.
